# EUROPÄISCHE PATENTSCHRIFT

(11) **EP 3 628 288 B1**
(45) Veröffentlichungstag und Bekanntmachung des Hinweises auf die Patenterteilung: **09.02.2022**
(21) Anmeldenummer: 19200449.7
(22) Anmeldetag: 30.09.2019
(51) Int. Cl.: A61F 13/00, A61F 13/02

(54) **WUNDAUFLAGE**
WOUND DRESSING
PANSEMENT

(30) Priorität: 30.09.2018 DE 102018007692
(43) Veröffentlichungstag der Anmeldung: 01.04.2020
(73) Patentinhaber: Folwarzny, Alexander, 63457 Hanau (DE)
(72) Erfinder: Folwarzny, Alexander, 63457 Hanau (DE)
(74) Vertreter: Stoffregen, Hans-Herbert

(56) Entgegenhaltungen:
- WO-A1-2010/092334
- WO-A1-2014/140608
- DE-A1-102011 050 047
- DE-U1-202007 019 565
- US-A- 3 529 597
- US-A1- 2011 213 287

## Beschreibung

Die Erfindung betrifft eine Wundauflage, umfassend eine saugfähige Schicht, wie Polyurethan-Schaumschicht, mit einer Oberseite, die zumindest bereichsweise mit einer Schutzschicht, wie semiokklusive Polyurethan-Folie, beschichtet ist und einer Unterseite, die vorzugsweise mit einer Haftschicht, wie adhäsive Silikon-Schicht, beschichtet ist.

Aus dem Stand der Technik sind Wundauflagen, insbesondere Schaumverbände für die Versorgung von exsudierenden Wunden bekannt, die z.B. unter der Bezeichnung SymbioFoam vertrieben werden. Die bekannten Wundauflagen umfassen einen absorbierenden Saugkörper, vorzugsweise in Form einer PU-Schaumschicht mit einer Oberseite, die mit einer Schutzschicht, vorzugsweise semiokklusiver Polyurethanfolie beschichtet ist. Eine Unterseite des Saugkörpers kann mit einer Haftschicht, insbesondere einer adhäsiven Silikonschicht, wie Silikon-Gitter oder Silikon-Pad, beschichtet sein.

Die aus dem Stand der Technik bekannten Wundauflagen sind als flächige, vorzugsweise rechteckige Auflage ausgebildet. Zur Behandlung von Wunden an Körperbereichen, wie Ferse, Ellenbogen, Brust, Achselhöhle oder Schulter mit gekrümmten Flächen sind die bekannten Wundauflagen oft nicht gut geeignet.

Die DE 10 2011 050 047 A1 betrifft einen Wundpflegeartikel für Kavitäten, umfassend mindestens eine flächige Lage, wobei die flächige Lage propellerförmig ausgebildet ist, mit mindestens zwei Flügeln und einem zentralen Bereich, wobei die Flügel um den zentralen Bereich des Propellers angeordnet sind und die Flügel über den zentralen Bereich miteinander verbunden sind. Ein Entfernen von Segmenten ist jedoch nicht vorgesehen.

Die DE 20 2007 019 565 U1 betrifft eine Wundauflage in Form eines klebefähigen Laminats, das eine entfernbare Abdeckung bildet, die Laschen aufweist, um ein Erfassen und Abziehen zu ermöglichen. Dabei kann die Wundauflage perforiert sein und zwar auch die Trennverkleidung. Die Perforation bietet eine Belüftung, ohne dass abzutrennende Segmente zur Verfügung gestellt werden sollen.

Die WO 97/21459 A1 betrifft eine Vorrichtung, die ein Pflaster umfasst, das hautseitig von einer einen Schlitz aufweisenden Folie abdeckbar ist. Ein Abtrennen von Segmenten einer saugfähigen Schicht erfolgt nicht.

Die EP 0 026 572 B1 betrifft eine Wundauflage mit mehreren Schichten, von denen eine ein Kohlenstoffgewebe sein kann.

Die US 3,529,597 B betrifft eine Fingerspitzenbandage, die aus mehreren durch Schlitze getrennten Abschnitten besteht. Ein Abtrennen von Segmenten ist nicht beschrieben.

Die DE 296 21 366 U1 bezieht sich auf eine Applikationshilfe für Verbände und zeichnet sich dadurch aus, dass eine entfernbare Stützfolie vorgesehen ist, die vor der Benutzung eine Polymerfolie und eine auf diese aufgebrachte aufklebende Schicht abdeckt. Die Schutzsicht weist Flügel auf, um ein Abziehen zu erleichtern.

Die DE 10 2008 062 472 A1 betrifft einen Wundverband, der für die Unterdrucktherapie bestimmt ist. Dabei weist der Verband eine Wundkontaktschicht mit Erhebungen und Vertiefungen auf, um mit einer Hautoberfläche im Bereich der Erhebungen in Kontakt zu gelangen.

Die WO 2014/14068 A1 betrifft Wundverbandsysteme zur Schaffung eines Hauptteils eines Wundverbandes für die Verwendung bei der Wundversorgung. Der Hauptteil des Wundverbandes ist insbesondere zum Abdichten eines beschneidbaren Verbandes ausgebildet, und umfasst einen Hauptteil eines Verbandes oder einer Zelle, der in Fluidverbindung mit zusätzlichen Verbandsteilen oder Zellen zur Verwendung in der Wundversorgung ist oder steht.

Die US 2011/213287 A1 betrifft Ausführungsformen, die auf die Behandlung von Wunden mit Unterdruck ausgerichtet sind. Die Ausführung umfasst ein Schaumstoffkissen, das für die Verwendung an abdominalen Wundstellen geeignet sein kann und dessen Größe dimensionsunabhängig ist.

Die WO 2010/092334 A1 bezieht sich auf ein Wundverpackungsmaterial, das zur Verwendung bei der Unterdruck-Wundtherapie geeignet ist und einen Körper aus einem porösen Material umfasst, wobei der Körper abreißbare Bereiche umfasst, die eine Vielzahl von Abschnitten definieren, wobei die abreißbaren Bereiche es ermöglichen, die Abschnitte selektiv aus dem Körper zu entfernen.

Davon ausgehend liegt der vorliegenden Erfindung die Aufgabe zugrunde, eine Wundauflage der eingangs genannten Art derart weiterzubilden, dass eine Applikation auf Körperbereiche mit stark gekrümmter Oberflächenkontur auf einfache Weise ermöglicht wird.

Die Aufgabe wird erfindungsgemäß dadurch gelöst, dass in der saugfähigen Schicht zumindest ein Schnitt und/oder zumindest eine perforierte Trenn- bzw. Reißlinie zur Ausbildung zumindest eines werkzeuglos aus der saugfähigen Schicht abtrennbaren Segmentes eingebracht ist und dass die saugfähige Schicht nach Abtrennen zumindest eines Segmentes aus der Schicht mittels eines von der Schutzschicht ausgehenden freistehenden Klebeabschnitts bei gleichzeitiger Abdeckung des zumindest einen Schnittes, der zumindest einen perforierten Trenn- bzw. Reißlinie und/oder zumindest eines in der Schicht verbleibenden Segmentes wasser- und bakteriendicht verschließbar ist.

Erfindungsgemäß ist in der saugfähigen Schicht zumindest ein Schnitt und/oder zumindest eine perforierte Trenn- bzw. Reißlinie zur Ausbildung zumindest eines werkzeuglos aus der saugfähigen Schicht abtrennbaren Segmentes eingebracht, wobei die saugfähige Schicht nach abtrennen zumindest eines Segmentes aus der Schicht mittels eines von der Schutzschicht ausgehenden freistehenden Klebeabschnitts bei gleichzeitiger Abdeckung des zumindest einen Schnittes, der zumindest einen perforierten Trenn- bzw. Reißlinie und/oder zumindest eines in der Schicht verbleibenden Segmentes, vorzugsweise wasser- und bakteriendicht, verschließbar ist.

Gegenüber dem Stand der Technik wird der Vorteil erreicht, dass die Wundauflage individuell durch abtrennen von Segmenten aus der saugfähigen Schicht auf einfache Weise an Körperbereiche mit stark gekrümmter Oberfläche angepasst werden kann. Durch den von der Schutzschicht ausgehenden freistehenden Klebeabschnitt wird sowohl eine Fixierung als auch ein vollflächiger Schutz vor Wasser und Bakterien erreicht.

Gemäß einer bevorzugten Ausführungsform ist die saugfähige Schicht kreisförmig ausgebildet, wobei sich der Schnitt in der Schicht, ausgehend von einem umlaufenden Rand, in Richtung eines Zentrums, wie Mittelpunktes, erstreckt, wobei in einem sich an den Schnitt in Umfangsrichtung anschließenden Kreissektor zur Bildung der abtrennbaren Segmente in Form von Kreissektorabschnitten die perforierten Trenn- bzw. Reißlinien ausgehend von dem umlaufenden Rand in Richtung des Mittelpunktes ausgebildet sind.

Vorzugsweise ist die Schutzschicht kreisförmig ausgebildet und im Bereich außerhalb der abtrennbaren Kreissektoren mit der Oberseite der saugfähigen Schicht verbunden, wie verklebt, wobei der freistehende Klebeabschnitt integraler Bestandteil der Schutzschicht ist und entlang einer parallel oder im Wesentlichen parallel zu dem Schnitt verlaufenden Biegelinie mit der Schutzschicht verbunden ist.

Eine weitere bevorzugte Ausführung zeichnet sich dadurch aus, dass die saugfähige Schicht kreisförmig ausgebildet und vollflächig mit der kreisförmig ausgebildeten Schutzschicht beschichtet ist, wobei der Schnitt sowie die perforierten Trenn- bzw. Reißlinien sowohl in der saugfähigen Schicht als auch in der Schutzschicht ausgebildet sind, wobei der freistehende Klebeabschnitt als separater Klebeabschnitt ausgebildet ist, umfassend eine Lasche, die auf einer Oberfläche der Schutzschicht entlang des Schnittes verbunden, wie aufgeklebt, ist, wobei sich von der Lasche entlang einer Biegelinie der Klebeabschnitt in Form einer Finne erstreckt.

Des Weiteren wird vorgeschlagen, dass die Schutzfolie zwei Abschnitte aufweist, wobei ein erster Abschnitt halbkreisförmig ausgebildet ist und vollflächig mit der Oberseite des Saugkörpers verbunden, wie verklebt, ist und wobei ein zweiter Abschnitt halbkreisförmig ausgebildet ist, und einen ersten Teilbereich aufweist, der mit der Oberseite verbunden, wie verklebt, ist und einen zweiten Teilbereich, der über eine Biegelinie mit dem ersten Teilbereich verbunden ist und den freistehenden Klebeabschnitt bildet.

Um eine wasser- und bakteriendichte Oberfläche zu erhalten ist vorgesehen, dass die Lasche mit Klebeabschnitt und/oder der zweite Abschnitt der Schutzschicht eine Radialerstreckung L aufweisen, die größer ist als ein Radius R der saugfähigen Schicht und/oder dass der erste und zweite Abschnitt entlang einer Diagonalen einen Überlappungsbereich bilden.

Der freistehende Klebeabschnitt weist bevorzugt jeweils eine Fläche auf, die größer ist als eine von den Kreissektorabschnitten aufgespannte Fläche.

Zur vereinfachten Applikation ist vorgesehen, dass der freistehende Klebeabschnitt eine Klebefläche aufweist, die mit einer Abdeckfolie abgedeckt ist.

Besonders bevorzugt ist vorgesehen, dass die saugfähige Schicht eine PU-Schaumschicht und/oder ein Hydropolymer- oder Natriumpolyacrylkissen ist und/oder dass die Schutzschicht und die freistehende Klebeschicht eine semiokklusive Polyurethan-Folie ist und/oder dass die Haftschicht ein adhäsives Silikon-Gitter ist oder adhäsive Silikon-Pads aufweist.

Zwischen der Unterseite der saugfähigen Schicht und der Haftschicht kann vorzugsweise ein Kohlenstoffgewebe angeordnet sein. Sowohl in der saugfähigen Schicht, dem Kohlenstoffgewebe sowie der Haftschicht können der Schnitt und/oder die perforierten Trenn- bzw. Reißlinien ausgebildet sein.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von den Zeichnungen zu entnehmenden bevorzugten Ausführungsbeispielen.

Es zeigen:
- Fig. 1: eine perspektivische Darstellung einer ersten Ausführungsform eines erfindungsgemäßen Schaumverbandes,
- Fig. 2: eine perspektivische Darstellung einer zweiten Ausführungsform eines erfindungsgemäßen Schaumverbandes,
- Fig. 3: eine Detailansicht einer Applikationshilfe der zweiten Ausführungsform gemäß Fig. 2,
- Fig. 4: eine perspektivische Darstellung einer dritten Ausführungsform eines erfindungsgemäßen Schaumverbandes,
- Fig. 5: eine Draufsicht der dritten Ausführungsform gemäß Fig. 4,
- Fig. 6: eine perspektivische Darstellung einer vierten Ausführungsform eines erfindungsgemäßen Schaumverbandes,
- Fig. 7: eine perspektivische Darstellung einer fünften Ausführungsform eines erfindungsgemäßen Schaumverbandes und
- Fig. 8: einen Schichtaufbau des erfindungsgemäßen Schaumverbandes gemäß Fig. 7.

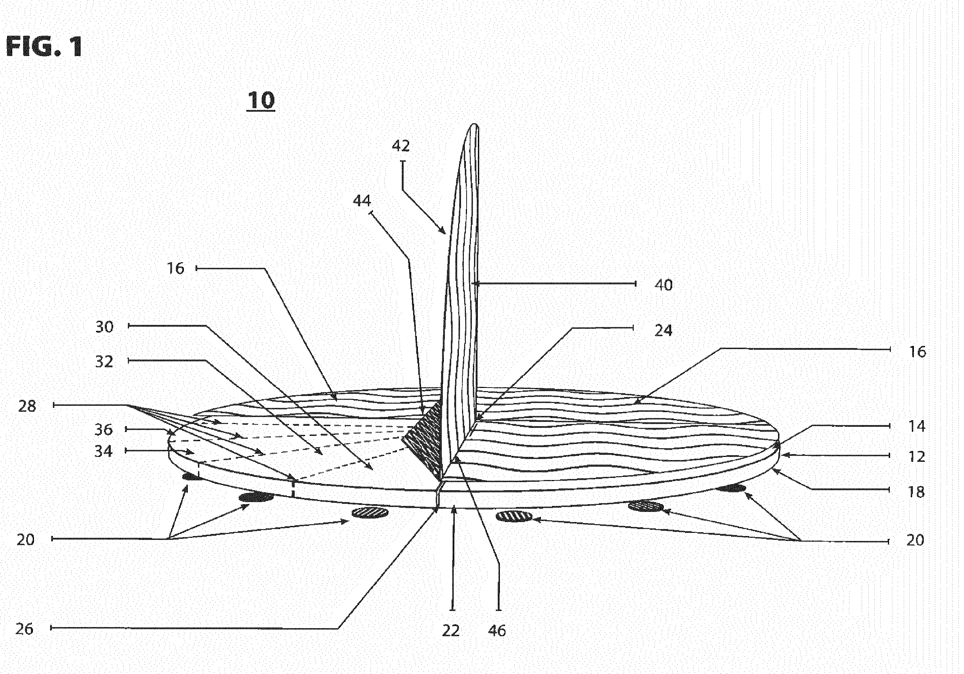

Fig. 1 zeigt in perspektivischer Ansicht eine erste Ausführungsform einer erfindungsgemäßen Wundauflage 10, umfassend einen Saugkörper in Form einer Polyurethan-Schaumschicht 12, deren Oberseite 14 zumindest bereichsweise mit einer Schutzschicht, wie wasser- und bakteriendichten, semiokklusiven Polyurethan-Folie 16, beschichtet ist. Eine Unterseite 18 der Polyurethan-Schaumschicht 12 weist zumindest abschnittsweise eine Haftschicht 20 in Form von adhäsiven Silikon-Pads auf.

Gemäß der Erfindung ist vorgesehen, dass die Polyurethan-Schaumschicht 12 kreisförmig ausgebildet ist, wobei sich von einem äußeren Rand 22 der Polyurethan-Schaumschicht 12 bis zu einem Mittelpunkt 24 in radialer Richtung ein Schnitt 26 und/oder eine vorzugsweise perforierte Trenn- oder Reißlinie 28 in der Polyurethan-Schaumschicht 12 ausgebildet ist.

In dem dargestellten Ausführungsbeispiel sind in einem in Umfangsrichtung an den Schnitt 26 angrenzenden Kreissektor ein oder mehrere vom äußeren Rand 22 zum Mittelpunkt 24 sich erstreckende, vorzugsweise perforierte Trennlinien bzw. Reißlinien 28 in den PU-Schaum 12 eingebracht. Durch die Trennlinien 28 werden einzelne Kreissektorabschnitte 30...36 gebildet, die werkzeuglos aus der PU-Schaumschicht 12 abgetrennt werden können. Nach Abtrennen eines der Kreissektorabschnitte kann aus der kreisförmig ebenen Wundauflage eine an eine gekrümmte Körperkontur angepasste kegelstumpfförmige Wundauflage ausgebildet werden.

Die Oberseite 14 des PU-Schaums 12 ist gemäß der in Fig. 1 dargestellten Ausführungsform im Bereich der außerhalb der abtrennbaren Kreissektorabschnitte 30... 36 mit der semiokklusiven Polyurethan-Folie 16 abgedeckt. Zur Abdeckung der Kreissektorabschnitte 30...36 weist die PU-Folie 16 einen freistehenden Folienabschnitt 40 in Form einer Finne mit rückseitiger Klebefläche 42 auf, die mit einer Abdeckfolie 44 versehen ist. Der Folienabschnitt 40 ist integraler Bestandteil der PU-Folie 16 und entlang einer Biegelinie 46 an der PU-Folie 16 angelenkt. Die Biegelinie 46 erstreckt sich parallel oder im Wesentlichen parallel zu dem Schnitt 26.

Betreffend die Herstellung der Wundauflage 10 gemäß Fig. 1 ist anzumerken, dass in einem ersten Verfahrensschritt die PU-Schaumschicht 12 in Form einer Kreisfläche hergestellt und anschließend oder gleichzeitig der Schnitt 26 sowie die Trenn- bzw. Reißlinien 28 zur Ausbildung der Kreissektorabschnitte 30...36 in die PU-Schaumschicht eingebracht werden. Sodann kann auf die Oberseite 14 des PU-Schaums 12 die PU-Folie 16 mit Folienabschnitt 40 aufgebracht, wie aufgeklebt, werden. Anschließend oder gleichzeitig kann die Haftschicht 20, im dargestellten Ausführungsbeispiel die adhäsiven Silikon-Pads 20, auf die Unterseite 18 der PU-Schaumschicht 12 aufgebracht, wie aufgeklebt, werden.

Betreffend die Verwendung der Wundauflage 10 ist anzumerken, dass entsprechend der zu behandelnden Wunde bzw. Körperstelle ein oder mehrere Kreissektorabschnitte 30, 32, 34, 36 der PU-Schaumschicht 12 abgetrennt werden können. Anschließend kann die flächige PU-Schaumschicht 12 zu einer kegelstumpfförmigen Fläche geformt werden, wobei eine Schnittkante des Schnittes 26 an einer Abrisskante einer Trenn- bzw. Reißlinie 28 anliegt. Sodann kann die Schutzfolie 44 von dem Klebeabschnitt 40 abgetrennt und der Klebeabschnitt 40 über die verbleibenden Kreissektorabschnitte 30...36 verklebt werden, so dass die Oberseite 14 der PU-Schaumschicht 12, insbesondere die Trenn- bzw. Reißlinien 28, lückenlos durch die PU-Folie 16 abgedeckt ist.

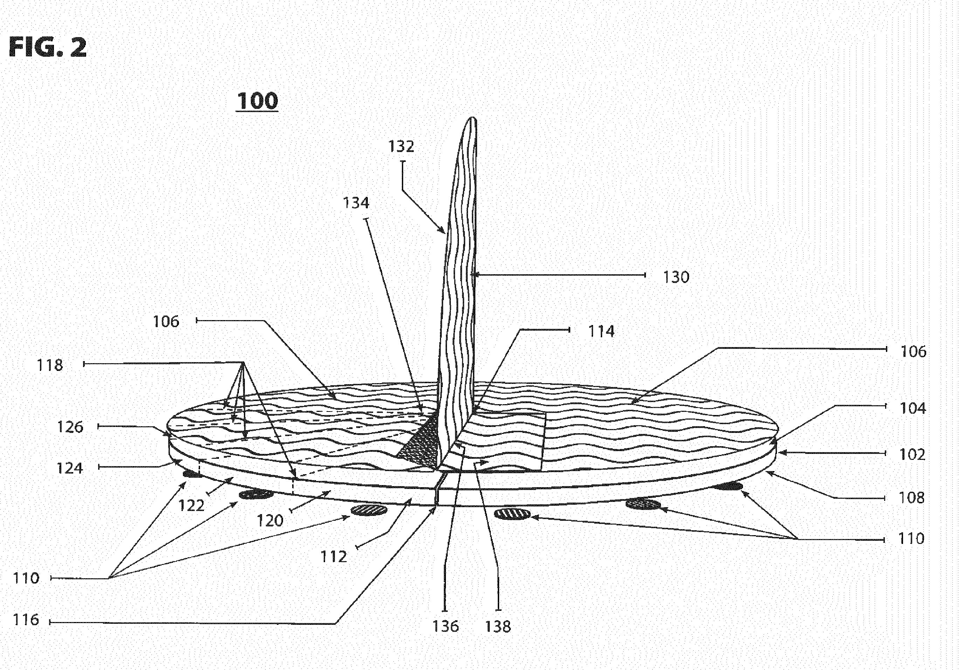

Fig. 2 zeigt eine zweite Ausführungsform einer Wundauflage 100, umfassend einen saugfähigen Körper in Form einer kreisförmigen PU-Schaumschicht 102, deren Oberseite 104 vollflächig mit einer Schutzschicht, wie einer semiokklusiven PU-Folie 106, beschichtet ist.

Eine Unterseite 108 ist abschnittsweise mit einer Haftschicht 110 beschichtet, die im dargestellten Ausführungsbeispiel eine Vielzahl von Silikon-Pads 110 aufweist, die in beliebiger Form auf der Unterseite 108 der PU-Schaumschicht 102 angeordnet sind.

Gemäß der Erfindung ist bei der Wundauflage 100 vorgesehen, dass ausgehend von einem umlaufenden Rand 112 bis zu einem Mittelpunkt 114 der PU-Schaumschicht 102 sowie der PU-Folie 106 in radialer Richtung zumindest ein Schnitt 116 und/oder zumindest eine vorzugsweise perforierte Trenn- bzw. Reißlinie 118 in der PU-Schaumschicht 102 verläuft. Des Weiteren sind in Umfangsrichtung weitere Trenn- bzw. Reißlinien 118 in der PU-Schaumschicht 102 sowie der PU-Folie 106 eingebracht, die sich ebenfalls von dem umlaufenden Rand 112 in Richtung bis zum Mittelpunkt 114 erstrecken. Dadurch werden Kreissektorabschnitte 120, 122, 124, 126, ausgebildet, die werkzeuglos aus der PU-Schaumschicht 102 abgetrennt werden können.

Die in Fig. 2 dargestellte Wundauflage 100 unterscheidet sich von der in Fig. 1 dargestellten Wundauflage 10 dadurch, dass die semiokklusive PU-Folie 106 vollflächig auf der Oberseite 104 der PU-Schaumschicht 102 aufgeklebt ist und folglich auch die perforierten Trenn- bzw. Reißlinien 118 bzw. den Schnitt 116 enthält. Auch bei dieser Ausführungsform können die Kreissektoren 120, 122, 124, 126 auf einfache Weise werkzeuglos abgetrennt werden, um die Wundauflage 100 an eine gekrümmte Körperkontur anzupassen.

Zur Abdeckung der perforierten Trenn- bzw. Reißlinien 118 ist bei der Wundauflage 100 erfindungsgemäß vorgesehen, dass entlang des Schnittes 116 ein separater freistehender Folienabschnitt 130 in Form einer Finne angeordnet ist. Der Folienabschnitt 130 aus einer semiokklusiven PU-Folie weist rückseitig eine Klebefläche 132 auf, die durch eine abtrennbare Abdeckfolie 134 geschützt ist. Der Folienabschnitt 130 ist über eine Biegelinie 136 mit einer Lasche 138 verbunden, wobei sich die Biegelinie 136 parallel oder im Wesentlichen parallel zu dem Schnitt 116 erstreckt. Die Lasche 138 weist unterseitig eine Klebefläche auf und ist mit der Oberseite der PU-Folie 106 verklebt.

Die Wundauflage 100 zeichnet sich insbesondere dadurch aus, dass die Fläche des Folienabschnitts 130 größer als die Fläche des Kreissektors ist, in dem die perforierten Trenn- bzw. Reißlinien 118 ausgebildet sind. Folglich kann die Wundauflage 100 ohne Einschränkung hinsichtlich der Wasser- und Bakteriendichtheit auch in einem flächigen, ebenen Zustand verwendet werden, d.h. wenn keine Kreissektorabschnitte ausgebrochen sind. Durch den Folienabschnitt 130 der PU-Folie 106 ist sichergestellt ist, dass die Oberfläche der PU-Schaumschicht 102 vollflächig abgedeckt ist.

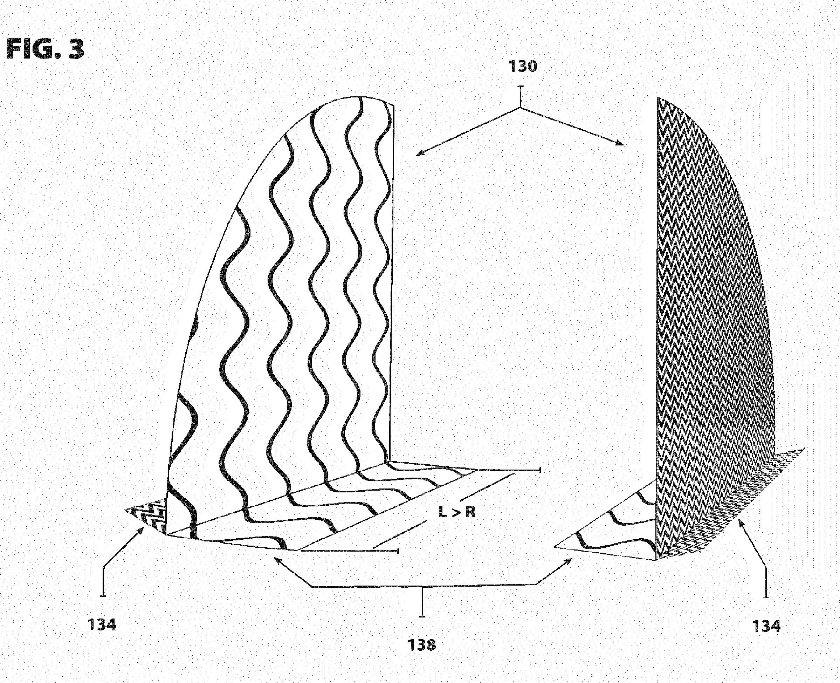

Fig. 3 zeigt eine Vorder- und Rückansicht des Folienabschnitts 130 in perspektivischer Darstellung. Die Klebelasche 138 ist vorzugsweise rechteckförmig ausgebildet und weist eine Länge L auf, die größer als ein Radius R der Wundauflage ist. Dadurch wird ein Überlappungsbereich zwischen PU-Folie 106 und Folienabschnitt 130 bei flächiger, ebener Anwendung der Wundauflage 100 sichergestellt. Alternativ kann vorgesehen sein, dass der Folienabschnitt 130 eine Umfangserstreckung aufweist, die größer ist als Umfangserstreckung der Kreissektorabschnitte 120, 122, 124, 126.

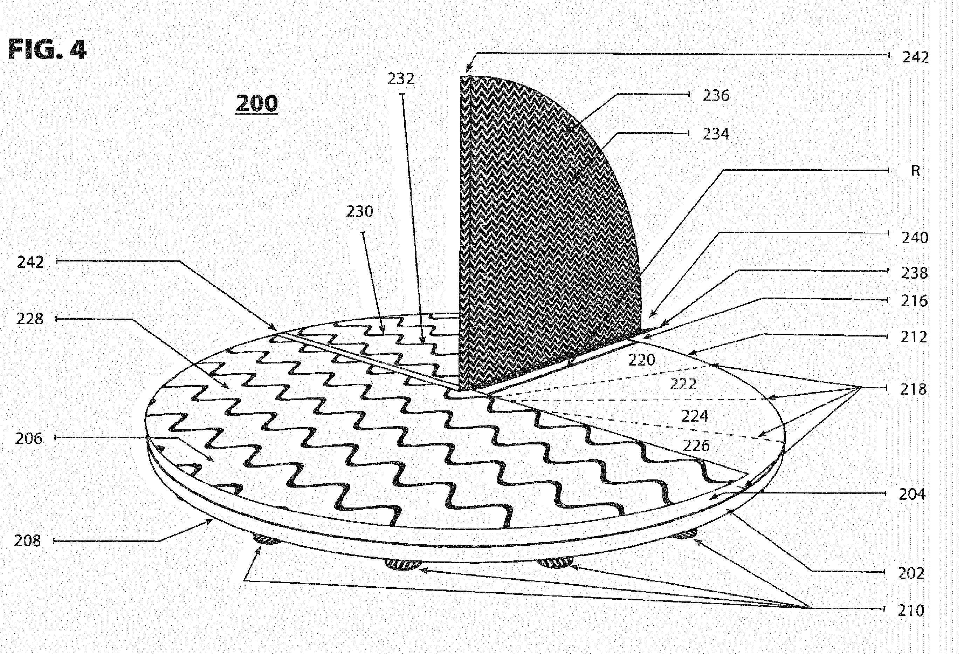

Fig. 4 zeigt in perspektivischer Darstellung eine dritte Ausführungsform einer Wundauflage 200. Die Wundauflage 200 umfasst einen Saugkörper 202 aus einer kreisförmigen PU-Schaumschicht mit einer Oberseite 204 auf der eine semiokklusive Polyurethan-Folie 206 aufgebracht, wie aufgeklebt, ist. Auf einer Unterseite 208 ist zumindest bereichsweise eine Haftschicht 210 in Form von adhäsiven Silikon-Pads aufgebracht.

Gemäß der Erfindung ist in der PU-Schaumschicht 202, ausgehend von einem umlaufenden Rand 212 bis zum Mittelpunkt 214, ein Schnitt 216 eingebracht. Ferner sind in Umfangsrichtung eine oder mehrere vorzugsweise perforierte Trennlinie/n 218 vorgesehen, die sich von dem umlaufenden Rand 212 in Richtung des Mittelpunktes 214 erstrecken und einen Kreissektor des PU-Schaumschicht 202 in werkzeuglos abtrennbare Kreissektorabschnitte 220...226, unterteilen. Die abtrennbaren Kreissektorabschnitte 220...226 sind nicht durch eine semiokklusive Polyurethan-Folie abgedeckt.

Gemäß der erfindungsgemäßen Ausführung der Wundauflage 200 ist vorgesehen, dass die semiokklusive Polyurethan-Folie 206 zwei Abschnitte aufweist, nämlich einen ersten Abschnitt 228, der vorzugsweise halbkreisförmig ausgebildet ist und eine erste Hälfte der kreisförmigen PU-Schaumschicht 202 abdeckt. Des Weiteren ist ein zweiter Abschnitt 230 vorgesehen, der ebenfalls halbkreisförmig ausgebildet ist, wobei ein erstes Teilstück 232 des zweiten Abschnitts 230 mit der Oberseite 204 verklebt ist und ein zweites Teilstück 234 einen freistehenden Folienabschnitt in Form einer Finne mit Klebefläche 236 und Abdeckfolie 238 bildet und über eine Biegelinie 240 mit dem ersten Teilstück 232 verbunden ist.

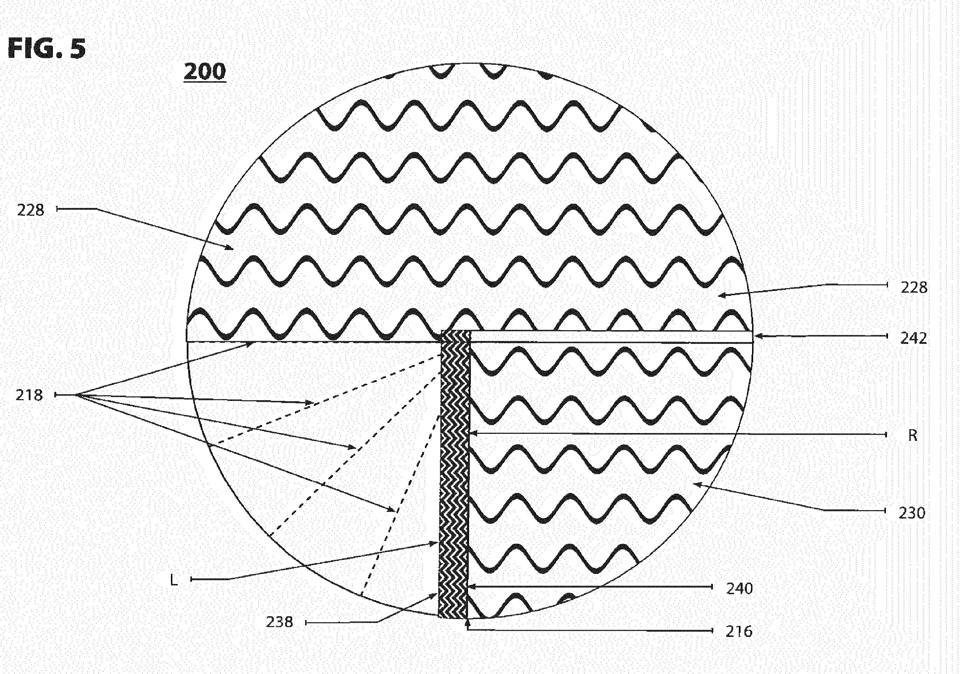

Fig. 5 zeigt eine Draufsicht der Wundauflage 200. Der zweite Abschnitt 230 der semiokklusiven Polyurethan-Folie weist eine Länge L auf, der größer ist als ein Radius R der PU-Schaumschicht, so dass im Bereich einer Stoßstelle zwischen dem ersten halbkreisförmigen Abschnitt 228 und dem ersten Teilstück 232 sowie dem zweiten Teilstück 234 ein Überlappungsbereich 242 entsteht, um eine vollständige Abdichtung der Oberseite der PU-Schaumschicht 202 zu erreichen.

Bei der Applikation des Wundverbandes 200 auf eine Wunde kann, wie auch bei den zuvor beschriebenen Ausführungsformen, ein Kreissektorabschnitt 220...226 werkzeuglos aus der PU-Schaumschicht 202 abgetrennt und sodann die Wundauflage 200 in entsprechende Form gebracht werden. Anschließend kann die Abdeckfolie 238 abgetrennt und das zweite Teilstück 234 in Form der Finne auf die verbleibenden Kreissektorabschnitte aufgeklebt werden, um eine geschlossene Oberfläche zu bilden.

Die Wundauflage 200 zeichnet sich auch dadurch aus, dass diese in ebener Form, d. h. ohne Abtrennen von Kreissektorabschnitten 220...226, verwendet werden kann und trotzdem eine ausreichende Wasser- und Bakteriendichtheit sichergestellt ist.

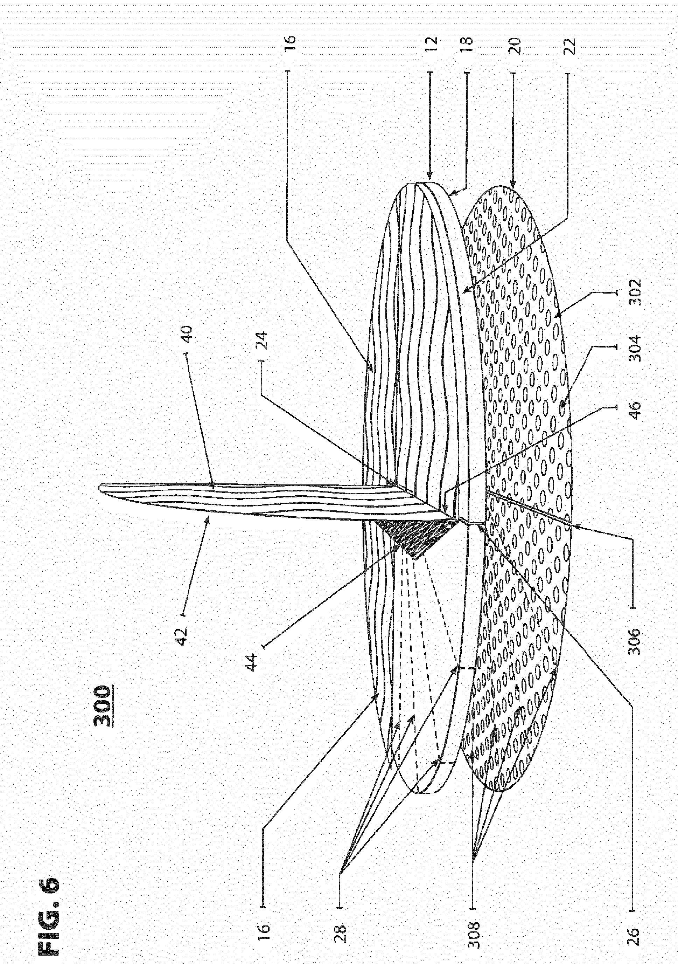

Fig. 6 zeigt eine vierte Ausführungsform einer Wundauflage 300, die im Wesentlichen der Ausführungsform der Wundauflage 10 entspricht, wobei die Haftschicht 20 in Form eines adhäsiven Silikon-Gitters 302 ausgebildet ist, welches entsprechend der Form der PU-Schaumschicht kreisförmig ausgebildet und mit der Unterseite 18 der PU-Schaumschicht 12 verbunden, wie verklebt, ist. In dem adhäsiven Silikon-Gitter 302 sind Durchbrechungen, wie Löcher 304, ausgebildet, wodurch ein Wundkontakt mit dem PU-Schaum 12 hergestellt wird. Des Weiteren sind in dem Silikon-Gitter in radialer Richtung ein Schnitt 306 sowie korrespondierend perforierte Trenn- bzw. Reißlinien 308 eingebracht. Vorzugsweise ist das adhäsive Silikon-Gitter 302 bereits mit dem PU-Schaum 12 verbunden, wobei anschließend der Schnitt 26 bzw. 306 sowie die Trenn- bzw. Reißlinien 28, 308 in einem gemeinsamen Verfahrensschritt eingebracht werden.

Die Applikation des Wundverbandes 300 erfolgt entsprechend der Applikation des Wundverbandes 10 gemäß Fig. 1.

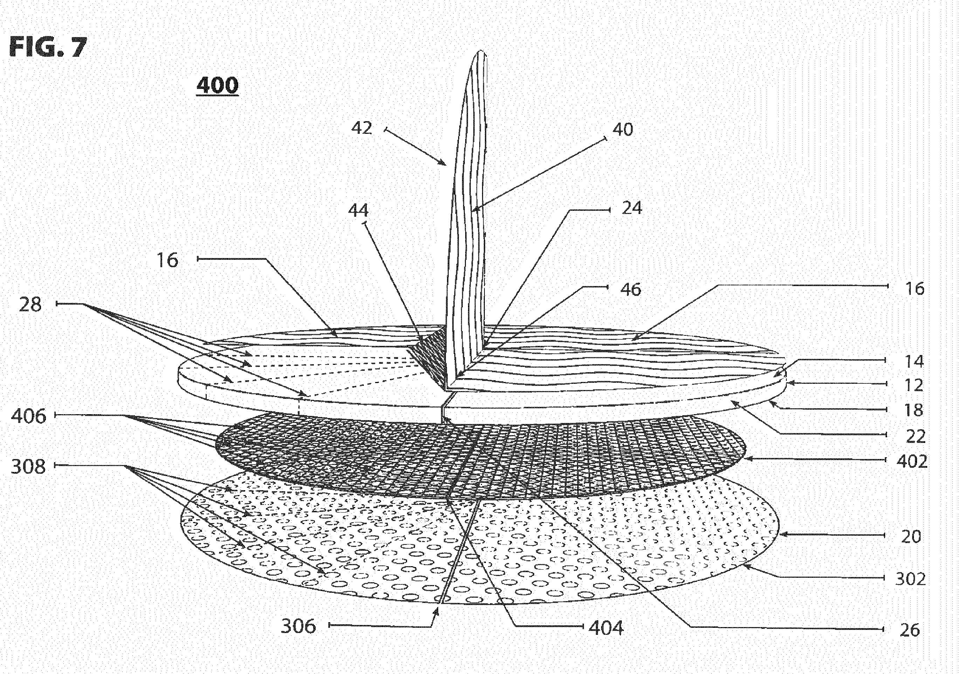

Fig. 7 zeigt in perspektivischer Darstellung eine fünfte Ausführungsform eines Wundverbandes 400. Der Wundverband 400 entspricht im Wesentlichen dem Wundverband 300 gem. Fig. 6, wobei zwischen der Haftschicht 20 und der Unterseite 18 der PU-Schaumschicht 12 ein Kohlenstoffgewebe 402 in gewebter oder gestrickter Form angeordnet ist. Im Sinne der Erfindung ist in dem Kohlenstoffgewebe 402 ebenfalls in radialer Richtung ein Schnitt 404 und/oder perforierte Trenn- bzw. Reißlinien 406 eingebracht.

Bei der Herstellung der Wundauflage 400 kann zunächst die PU-Schaumschicht 12, das Kohlenstoffgewebe 402 sowie das adhäsive Silikon-Gitter 302 als Sandwich miteinander verbunden und anschließend der Schnitt 26, 306, 404 und/oder die perforierten Trenn- bzw. Reißlinien 28, 308, 406 eingebracht werden. Sodann kann die semiokklusive Polyurethan-Folie 16 mit Folienabschnitt 40 auf die Oberseite 14 des PU-Schaums 12 aufgeklebt werden.

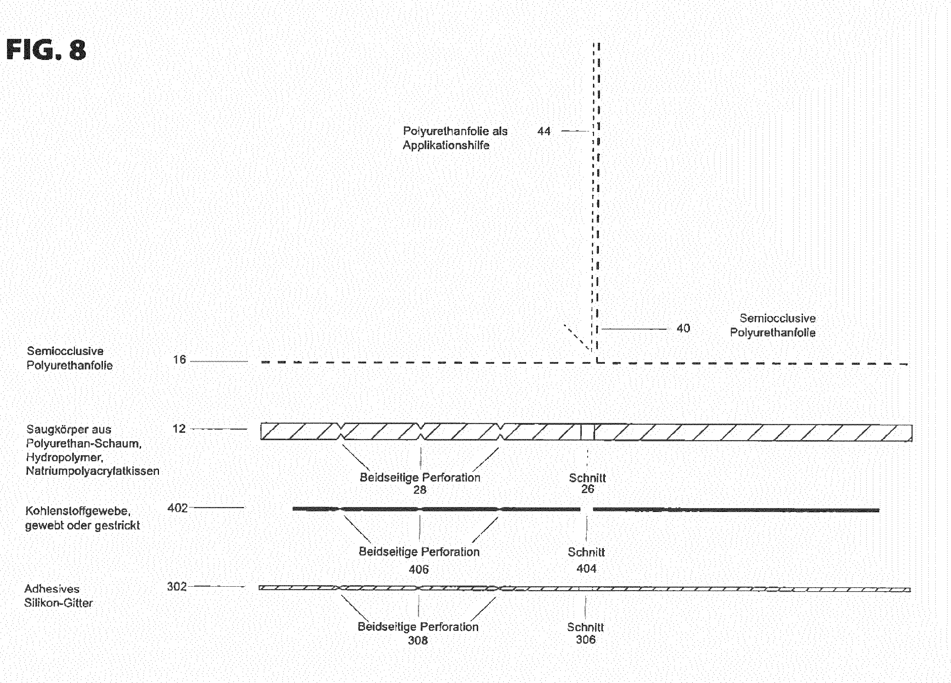

Fig. 8 zeigt rein schematisch einen Schichtaufbau der Wundauflage 400 in Explosionsdarstellung.

## Patentansprüche

1. Wundauflage (10, 100, 200, 300, 400) umfassend eine saugfähige Schicht (12, 102, 202), wie Polyurethan-Schaumschicht, mit einer Oberseite (14, 104, 204), die zumindest bereichsweise mit einer Schutzschicht (16, 106, 206), wie semiokklusive Polyurethan-Folie, beschichtet ist und einer Unterseite (18, 108, 208), die vorzugsweise mit einer Haftschicht (20, 210, 302), wie adhäsive Silikon-Schicht beschichtet ist,
**dadurch gekennzeichnet,**
**dass** in der saugfähigen Schicht (12, 102, 202) zumindest ein Schnitt (26, 116, 216) und/oder zumindest eine perforierte Trenn- bzw. Reißlinie (28, 118, 218) zur Ausbildung zumindest eines werkzeuglos aus der saugfähigen Schicht (12, 102, 202) abtrennbaren Segmentes (30, 32, 34, 36; 120, 122, 124, 126; 220, 222, 224, 226) eingebracht ist und dass die saugfähige Schicht (12, 102, 202) nach Abtrennen zumindest eines Segmentes ( 30, 32, 34, 36; 120, 122, 124, 126; 220, 222, 224, 226) aus der Schicht mittels eines von der Schutzschicht (16, 106, 206) ausgehenden freistehenden Klebeabschnitts (40, 130, 234) bei gleichzeitiger Abdeckung des zumindest einen Schnittes (26, 116, 216), der zumindest einen perforierten Trenn- bzw. Reißlinie (28, 118, 218) und/oder zumindest eines in der Schicht verbleibenden Segmentes ( 30, 32, 34, 36; 120, 122, 124, 126; 220, 222, 224, 226) wasser- und bakteriendicht verschließbar ist.

2. Wundauflage nach Anspruch 1,
**dadurch gekennzeichnet,**
**dass** die saugfähige Schicht (12, 102, 202) kreisförmig ausgebildet ist, wobei sich der Schnitt (26, 116, 216) in der Schicht (12, 102, 202) ausgehend von einem umlaufenden Rand (22, 112, 212) in Richtung eines Zentrums, wie Mittelpunktes (24, 114, 214), erstreckt und dass in einem sich an den Schnitt (26, 116, 216) in Umfangsrichtung anschließenden Kreissektor zur Bildung der abtrennbaren Segmente in Form von Kreissektorabschnitten (30, 32, 34, 36 120, 122, 124, 126; 220, 222, 224, 226) die perforierten Trenn- bzw. Reißlinien (28, 118, 218) ausgehend von dem umlaufenden Rand (22, 112, 212) in Richtung des Mittelpunktes (24, 114, 214) ausgebildet sind.

3. Wundauflage nach Anspruch 1 oder 2,
**dadurch gekennzeichnet,**
**dass** die Schutzschicht (16) kreisförmig ausgebildet und im Bereich außerhalb der abtrennbaren Kreissektoren (30...36) mit der Oberseite (14) der saugfähigen Schicht (12) verbunden, wie verklebt, ist, und dass der freistehende Klebeabschnitt (40) integraler Bestandteil der Schutzschicht (16) ist und entlang einer parallel oder im Wesentlichen parallel zu dem Schnitt (26) verlaufenden Biegelinie (46) mit der Schutzschicht (16) verbunden ist.

4. Wundauflage nach Anspruch 1 oder 2,
**dadurch gekennzeichnet,**
**dass** die saugfähigen Schicht (102) kreisförmig ausgebildet und vollflächig mit der kreisförmig ausgebildeten Schutzschicht (106) beschichtet ist, wobei der Schnitt (116) sowie die perforierten Trenn- bzw. Reißlinien (118) sowohl in der saugfähigen Schicht (102) als auch in der Schutzschicht (106) ausgebildet sind, wobei der freistehende Klebeabschnitt (130) als separater Klebeabschnitt ausgebildet ist, umfassend eine Lasche (138), die auf einer Oberfläche der Schutzschicht (106) entlang des Schnittes (116) verbunden wie aufgeklebt ist, wobei sich von der Lasche (138) entlang einer Biegelinie (136) der Klebeabschnitt (130) in Form einer Finne (130) erstreckt.

5. Wundauflage nach zumindest einem der vorhergehenden Ansprüche,
**dadurch gekennzeichnet,**
**dass** die Schutzfolie (206) zwei Abschnitte (228, 230) aufweist, wobei ein erster Abschnitt (228) halbkreisförmig ausgebildet ist und vollflächig mit der Oberseite (204) des Saugkörpers (202) verbunden, wie verklebt, ist und wobei ein zweiter Abschnitt (230) halbkreisförmig ausgebildet ist, und einen ersten Teilbereich (232) aufweist, der mit der Oberseite (204) verbunden, wie verklebt, ist und einen zweiten Teilbereich (234), der über eine Biegelinie (240) mit dem ersten Teilbereich (230) verbunden ist und den freistehenden Klebeabschnitt (234) bildet.

6. Wundauflage nach zumindest einem der vorhergehenden Ansprüche,
**dadurch gekennzeichnet,**
**dass** die Lasche (138) mit Klebeabschnitt (130) und/oder der zweite Abschnitt (228, 230) der Schutzschicht (206) eine Radialerstreckung L aufweisen, die größer ist als ein Radius RS der saugfähigen Schicht (102) und/oder dass der erste und zweite Abschnitt (228, 230) entlang einer Diagonalen einen Überlappungsbereich (242) bilden.

7. Wundauflage nach zumindest einem der vorhergehenden Ansprüche,
**dadurch gekennzeichnet,**
**dass** der freistehende Klebeabschnitt (40, 130, 234) jeweils eine Fläche aufweist, die größer ist als eine von den Kreissektorabschnitten (30, 32, 34, 36; 120, 122, 124, 126; 220, 222, 224, 226) aufgespannte Fläche.

8. Wundauflage nach zumindest einem der vorhergehenden Ansprüche,
**dadurch gekennzeichnet,**
**dass** der freistehende Klebeabschnitt (40, 130, 234) eine Klebefläche (42, 132, 236) aufweist, die mit einer Abdeckfolie (44, 134, 238) abgedeckt ist.

9. Wundauflage nach zumindest einem der vorhergehenden Ansprüche,
**dadurch gekennzeichnet,**
**dass** die saugfähige Schicht (12, 102, 202) eine PU-Schaumschicht ist und/oder dass die Schutzschicht (16, 106, 206) und die freistehende Klebeschicht (40, 130, 234) eine semiokklusive Polyurethan-Folie ist und/oder dass die Haftschicht (20, 110, 210, 302) ein adhäsives Silikon-Gitter (302) ist oder adhäsive Silikon-Pads (20) aufweist.

10. Wundauflage nach zumindest einem der vorhergehenden Ansprüche,
**dadurch gekennzeichnet,**
**dass** zwischen einer Unterseite (18) der saugfähigen Schicht (12) und der Haftschicht (20) ein Kohlenstoffgewebe (402) angeordnet ist.

11. Wundauflage nach Anspruch 10,
**dadurch gekennzeichnet,**
**dass** sowohl in der saugfähigen Schicht (12), dem Kohlenstoffgewebe (402) sowie der Haftschicht (20) der Schnitt (26, 404, 306) sowie die perforierten Trenn- bzw. Reißlinien (28, 406, 308) ausgebildet sind.

## Claims

1. Wound dressing (10, 100, 200, 300, 400) comprising an absorbent layer (12, 102, 202), such as a polyurethane foam layer, with an upper side (14, 104, 204) which is covered at least in some areas with a protective layer (16, 106, 206), such as a semi-occlusive polyurethane film, and with an underside (18, 108, 208), which is covered preferably with an adhesive layer (20, 210, 302), such as an adhesive silicone layer,
wherein
at least one incision (26, 116, 216) and/or at least one perforated dividing/tearing line (28, 118, 218) is provided in the absorbent layer (12, 102, 202) to form at least one segment (30, 32, 34, 36; 120, 122, 124, 126; 220, 222, 224, 226) separable without tools from said absorbent layer (12, 102, 202), and said absorbent layer (12, 102, 202) is sealable water-tight and bacteria-proof after separation of at least one segment (30, 32, 34, 36; 120, 122, 124, 126; 220, 222, 224, 226) from the layer by means of a protruding adhesive section (40, 130, 234) extending from the protective layer (16, 106, 206) with simultaneous covering of the at least one incision (26, 116, 216), of the at least one perforated dividing/tearing line (28, 118, 218) and/or of at least one segment (30, 32, 34, 36; 120, 122, 124, 126; 220, 222, 224, 226) remaining in the layer.

2. Wound dressing according to claim 1,
**wherein**
the absorbent layer (12, 102, 202) is designed circular, where the incision (26, 116, 216) in said layer (12, 102, 202) starting from an all-round edge (22, 112, 212) extends in the direction of a centre, such as a centre point (24, 114, 214), and the perforated dividing/tearing lines (28, 118, 218) starting from said all-round edge (22, 112, 212) in the direction of the centre point (24, 114, 214) are provided in a circle sector adjoining the incision (26, 116, 216) in the circumferential direction to form separable segments in the form of circle sector sections (30, 32, 34, 36 120, 122, 124, 126; 220, 222, 224, 226).

3. Wound dressing according to claim 1 or 2,
**wherein**
the protective layer (16) is designed circular and is connected, such as bonded, in the area outside the separable circle sectors (30...36) to the upper side (14) of the absorbent layer (12), and the protruding adhesive section (40) is an integral part of said protective layer (16) and is connected to said protective layer (16) along a bending line (46) running parallel or substantially parallel to the incision (26).

4. Wound dressing according to claim 1 or 2,
**wherein**
the absorbent layer (102) is designed circular and is covered over its full surface with the protective layer (106) of circular design, where the incision (116) and the perforated dividing/tearing lines (118) are provided both in the absorbent layer (102) and in said protective layer (106), where the protruding adhesive section (130) is designed as a separate adhesive section comprising a tab (138) which is connected, such as bonded, to a surface of said protective layer (106) along the incision (116), where said adhesive section (130) extends from the tab (138) in the form of a fin (130) along a bending line (136).

5. Wound dressing according to at least one of the preceding claims,
**wherein**
the protective film (206) has two sections (228, 230), where a first section (228) is designed semi-circular and is connected, such as bonded, to the upper side (204) of the absorbent body (202) over the full surface and a second section (230) is designed semi-circular and has a first partial area (232) which is connected, such as bonded, to the upper side (204) and a second partial area (234) which is connected via a bending line (240) to said first partial area (230) and forms the protruding adhesive section (234).

6. Wound dressing according to at least one of the preceding claims,
**wherein**
the tab (138) with adhesive section (130) and/or the second section (228, 230) of the protective layer (206) have a radial extent L which is greater than a radius RS of the absorbent layer (102), and/or the first and second sections (228, 230) form an overlap area (242) along a diagonal.

7. Wound dressing according to at least one of the preceding claims,
**wherein**
the protruding adhesive section (40, 130, 234) has in each case a surface which is greater than a surface created by the circle sector sections (30, 32, 34, 36; 120, 122, 124, 126; 220, 222, 224, 226).

8. Wound dressing according to at least one of the preceding claims,
**wherein**
the protruding adhesive section (40, 130, 234) has an adhesive surface (42, 132, 236) which is covered by a backing film (44, 134, 238).

9. Wound dressing according to at least one of the preceding claims,
**wherein**
the absorbent layer (12, 102, 202) is a PU foam layer and/or the protective layer (16, 106, 206) and the protruding adhesive layer (40, 130, 234) is a semi-occlusive polyurethane film, and/or the adhesive layer (20, 110, 210, 302) is an adhesive silicone grid (302) or has adhesive silicone pads (20).

10. Wound dressing according to at least one of the preceding claims,
**wherein**
a carbon tissue (402) is arranged between an underside (18) of the absorbent layer (12) and the adhesive layer (20).

11. Wound dressing according to claim 10,
**wherein**
the incisions (26, 404, 306) and the perforated dividing/tearing lines (28, 406, 308) are provided in the absorbent layer (12), in the carbon tissue (402) and in the adhesive layer (20).

## Revendications

1. Pansement (10, 100, 200, 300, 400) comprenant une couche absorbante (12, 102, 202) ainsi qu'une couche de mousse de polyuréthane avec une face supérieure (14, 104, 204) qui est revêtue au moins partiellement d'une couche protectrice (16, 106, 206), comme du film polyuréthane semi-occlusif et une face inférieure (18, 108, 208) revêtue de préférence d'une couche adhésive (20, 210, 302) telle qu'une couche adhésive de silicone. **caractérisé en ce**
**que** dans la couche absorbante (12, 102, 202) se trouve au moins une incision (26, 116, 216) et/ou au moins une ligne de séparation ou de déchirement perforée (28, 118, 218) pour constituer au moins un segment détachable (30, 32, 34, 36 ; 120, 122, 124, 126 ; 220, 222, 224, 226) de la couche absorbante (12, 102, 202), sans outil et qu'après détachement d'au moins un segment (30, 32, 34, 36 ; 120, 122, 124, 126 ; 220, 222, 224, 226) de la couche au moyen d'une section collante libre (40, 130, 234), partant de la couche protectrice (16, 106, 206) en recouvrant simultanément ladite au moins une incision (26, 116, 216), ladite au moins une ligne de séparation ou de déchirement perforée (28, 118, 218) et/ou au moins un segment (30, 32, 34, 36 ; 120, 122, 124, 126 ; 220, 222, 224, 226) restant dans la couche est refermable de manière étanche à l'eau et aux bactéries.

2. Pansement selon la revendication 1,
**caractérisé en ce**
**que** la couche absorbante (12, 102, 202) est conçue sous forme de cercle, sachant que l'incision (26, 116, 216) s'étend dans la couche (12, 102, 202) en partant d'un bord périphérique (22, 112, 212) en direction d'un centre tel que le point central (24, 114, 214) et que, dans un secteur de cercle se rattachant à l'incision (26, 116, 216) dans le sens circonférentiel pour former des segments détachables sous forme de parties de secteurs de cercle (30, 32, 34, 36 ; 120, 122, 124, 126 ; 220, 222, 224, 226), les lignes de séparation ou de déchirement perforées (28, 118, 218) sont constituées de manière à partir du bord périphérique (22, 112, 212) en direction du point central (24, 114, 214).

3. Pansement selon la revendication 1 ou 2,
**caractérisé en ce**
**que** la couche protectrice (16) est conçue sous forme de cercle et est reliée, telle que collée, à la face supérieure (14) de la couche absorbante (12) dans la zone située à l'extérieur des secteurs de cercle détachables (30...36), et que la section collante libre (40) fait partie intégrante de la couche protectrice (16) et est reliée à la couche protectrice (16) le long d'une ligne de pliage (46) s'étendant parallèlement ou essentiellement parallèlement à l'incision (26).

4. Pansement selon la revendication 1 ou 2,
**caractérisé en ce**
**que** la couche absorbante (102) est conçue sous forme de cercle et est revêtue sur toute sa surface d'une couche protectrice (106) conçue sous forme de cercle, sachant que l'incision (116) ainsi que les lignes de séparation et de déchirement perforées (118) sont constituées aussi bien dans la couche absorbante (102) que dans la couche protectrice (106), sachant que la section collante libre (130) est conçue sous forme de section collante séparée, comprenant une languette (138) qui est reliée, telle que collée sur une surface de la couche protectrice (106) le long de l'incision (116), sachant que la section collante (130) s'étend sous forme d'aileron (130) depuis la languette (138) le long d'une ligne de pliage (136).

5. Pansement selon au moins une des revendications précédentes,
**caractérisé en ce**
**que** le film protecteur (206) présente deux sections (228, 230), sachant qu'une première section (228) est conçue sous forme de demi-cercle et est reliée, telle que collée, sur toute sa surface à la face supérieure (204) du corps absorbant (202) et sachant qu'une seconde section (230) est conçue sous forme de demi-cercle et présente une première zone partielle (232) qui est reliée, telle que collée, à la face supérieure (204) et une seconde zone partielle (234) qui est reliée à la première zone partielle (230) via une ligne de pliage (240) et forme la section collante libre (234).

6. Pansement selon au moins une des revendications précédentes.
**caractérisé en ce**
**que** la languette (138) avec section collante (130) et/ou la seconde section (228, 230) de la couche protectrice (206) présente une extension radiale L qui est plus grande qu'un rayon RS de la couche absorbante (102) et/ou que la première et la seconde sections (228, 230) forment une zone de chevauchement (242) le long d'une diagonale.

7. Pansement selon au moins une des revendications précédentes, **caractérisé en ce**
**que** la section collante libre (40, 130, 234) présente respectivement une surface qui est plus grande qu'une surface sous-tendant les parties de secteurs de cercle (30, 32, 34, 36 ; 120, 122, 124, 126; 220, 222, 224, 226).

8. Pansement selon au moins une des revendications précédentes,
**caractérisé en ce**
**que** la section collante libre (40, 130, 234) présente une surface collante (42, 132, 236) qui est recouverte d'un film de recouvrement (44, 134, 238).

9. Pansement selon au moins une des revendications précédentes,
**caractérisé en ce**
**que** la couche absorbante (12, 102, 202) est une couche de mousse PU et/ou que la couche protectrice (16, 106, 206) et la couche collante libre (40, 130, 234) sont un film de polyuréthane semi-occlusif et/ou que la couche adhésive (20, 110, 210, 302) est un filet adhésif en silicone (302) ou présente des patins adhésifs en silicone (20).

10. Pansement selon au moins une des revendications précédentes,
**caractérisé en ce**
**qu'**un tissu de carbone (402) est disposé entre une face inférieure (18) de la couche absorbante (12) et la couche adhésive (20).

11. Pansement selon la revendication 10,
**caractérisé en ce**
**que** l'incision (26, 404, 306) ainsi que les lignes de séparation et de déchirement perforées (28, 406, 308) sont constituées aussi bien dans la couche absorbante (12), que dans le tissu de carbone (402) et que dans la couche adhésive (20).
